# EUROPEAN PATENT APPLICATION

(11) **EP 0 619 984 A1**
(43) Date of publication of application: **19.10.1994**
(21) Application number: 94302509.8
(22) Date of filing: 08.04.1994
(51) Int. Cl.: A61B 17/06

(54) **Surgical needle**

(30) Priority: 15.04.1993 JP 111156/93
(71) Applicant: KABUSHIKI KAISHA MATSUTANI SEISAKUSHO, Tochigi-Ken (JP)
(72) Inventor: Matsutani, Katja, Shioya-gun, Tochigi-ken (JP); Mashiko, Masaki, Shioya-gun, Tochigi-ken (JP); Saitoh, Akira, Shioya-gun, Tochigi-ken (JP)
(74) Representative: Pattullo, Norman

(57) **Abstract**

A surgical needle for sutural treatment having a body and a blunt needle point has a blunt ratio of special range. The blunt ratio is defined as a ratio of diameter D2 of the needle point to diameter D1 of the body and set in a range between 0.04 and 0.30. The surgical needle having special blunt ratio is capable of preventing health providers from hurting themselves by mistake even if the needle contacts the tissues of the health providers and of providing a property that once the needle enters the tissues the needle passes through the tissues smoothly.

## Description

### CROSS-REFERENCE TO RELATED APPLICATION

This application claims priority benefits under 35 U.S.C §119 of Japanese application Serial No. 5-111,156, filed April 15 th, 1993, the entire disclosure of which is incorporated herein by reference.

### BACKGROUND OF THE INVENTION

### 1. Field of the Invention

This invention relates to a surgical needle for a suture and, more particularly, to a surgical needle having a blunt point.

### 2. Description of Related Art

As conventional surgical needles, known are needles such as disclosed in Japanese Utility Model Publication, No. Showa-63-34,649 and Japanese Patent Publication, No. Heisei-5-18,576, and such as disclosed, as needles having a triangle cross section, in Japanese Utility Model Publication, No. Showa-62-23,457 and Japanese Patent Publication, No. Heisei-1-54,075. Many inventions are thus accomplished for reducing resistance against penetration of surgical needles when passed through the tissues.

Problems of infections among health providers and patients involved in infectious diseases, such as hepatitis, AIDS, MRSA (Methicillin-Resistant Staphylococcus Aureus), and whatever, are raised as major social problems these days. Such infections may happen by way of fluid, such as blood or the like, as carriers when health providers, such as doctors or the like, hurts themselves by mistake with the medical instrument. Hence, a needle with a sharp point may hurt health providers by themselves, and the health providers must pay close attention to handle the needle since in such situations the health providers have to take tremendous risks of infection between them, so that the health providers have to endure enormous mental pressure upon such risks.

On the other hand, in order to increase entry resistance of a surgical needle at a time that the point of the needle is pushed onto a skin, a blunt point of the needle is desirable. To the contrary, the blunt point can increase penetration resistance when the needle passes the tissues, so that the needle becomes difficult to handle.

### SUMMARY OF THE INVENTION

It is an object of the invention to provide a safe surgical needle hardly to hurt a health provider even if the provider handles it in a wrong manner and thereby to solve the problems happening in association with medical circumstances these days.

The foregoing object is accomplished with a surgical needle having a body taperingly extending and a needle point provided at one end of the body and formed to be blunt so that the needle point does not enter into tissues unless predetermined force or above is applied thereto. The body and the needle point are formed so as to satisfy a ratio of diameter D2 of the needle point to diameter D1 of the body in a range between 0.04 and 0.30. The surgical needle having the ratio (D2/D1) in a range between 0.04 and 0.30 can have relatively large resistance against the tissues when entering the tissues, and can be easily handled and controlled after the needle point has been entered into the tissues because of small penetration resistance at a time that the surgical needle passes through the tissues.

### BRIEF DESCRIPTION OF THE DRAWINGS

The above and other objects and features of the invention are apparent to those skilled in the art from the following preferred embodiments thereof when considered in conjunction with the accompanied drawings, in which:
Fig. 1 is a side view showing, partially broken away, a surgical needle according to a preferred embodiment of the invention;
Figs. 2(a) to 2(e) are side views showing essential portions of blunt needle points, respectively, according to the embodiment in Fig. 1;
Figs. 3 to 7 are graphs respectively showing penetration resistance measured in use of the needles shown in Figs 2(a) to 2(e);
Fig. 8 is a graph showing a relation between blunt ratio and load of peak X; and
Figs. 9(a) to 9(c) are side views showing a variety of point shapes of surgical needles according to the invention.

### DETAILED DESCRIPTION OF THE PREFERRED EMBODIMENTS

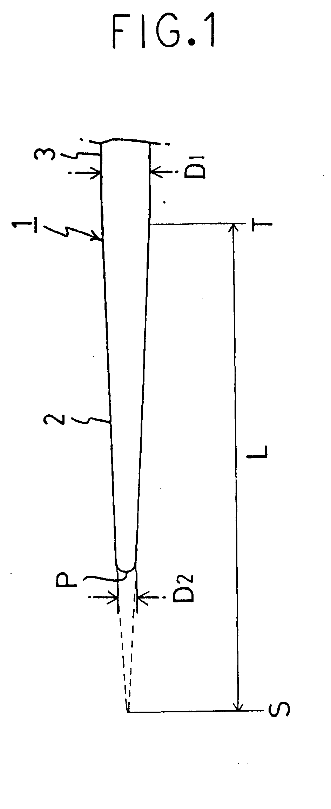

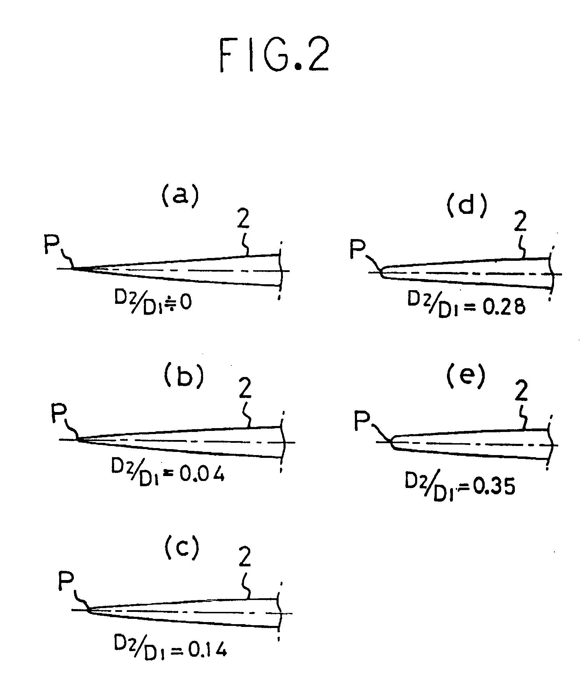

Referring to the drawings in detail, in particular, to Figs. 1, 2, a surgical needle according to a preferred embodiment of the invention is shown as designated by numeral 1.

The surgical needle 1 has a round cross section and a body extending straight. As shown in Fig. 1, a needle point 2 of the surgical needle 1 is formed in a taper shape getting slender as reaching further on the tip side thereof. A tip P of the needle point 2 is furnished to be round and blunt so that the needle point 2 does not enter into tissues unless a predetermined force or above is applied to the needle.

Taper ratio is indicated by how many times of the diameter D1 of the surgical needle 1 is equal to the length L from an inclination beginning point T to an imaginary end point S. For example, if the length L is five times longer of the diameter D1, the taper ratio is 5D; if seven times longer, the ratio is 7D. The ratio would be 3D to 9D in ordinary surgical needles.

Where the diameter of the body 3 of the surgical needle 1 is D1 and where the diameter of the tip P of the needle point 2 is D2, the ratio between them (D2/D1) is defined as a blunt ratio. Although a roundness given at the tip P of the needle point 2 is continuous with the tapered contour of the needle point 2, each point has its curve, and the diameter D2 of the needle point 2 is defined at a position where the curve changes greatly.

Method for measuring entering resistance when the surgical needle 1 is pushed onto a skin is described as follows. For measurement, so-called Porvair, generally a polyurethane sheet, is used. Porvair is a synthetic resin composed of a relatively hard, thin surface layer and a relatively soft, thick flesh layer, and its structure and nature are similar to that of human being's tissues. In order to measure entering resistance, the surgical needle 1 equipped with a pressure sensor is vertically moved down from the top surface of Porvair.

Various surgical needles 1 having its blunt ratio (D2/D1) are used for such measurements. That is, Fig. 2(a) shows a needle point 2, whose tip is made to be sharp, having the blunt ratio of nearly zero; Fig. 2(b) shows one having the blunt ratio of 0.04; Fig. 2(c) shows one having the blunt ratio of 0.14; Fig. 2(d) shows one having the blunt ratio of 0.23; and Fig. 2(e) shows one having the blunt ratio of 0.35. These needle points 2 are made from the same original needle having the taper ratio of 9D and ground gradually to form respective shapes shown in Figs. 2(a) to 2(e), so that slight differences of shapes among surgical needles do not bring experimental errors.

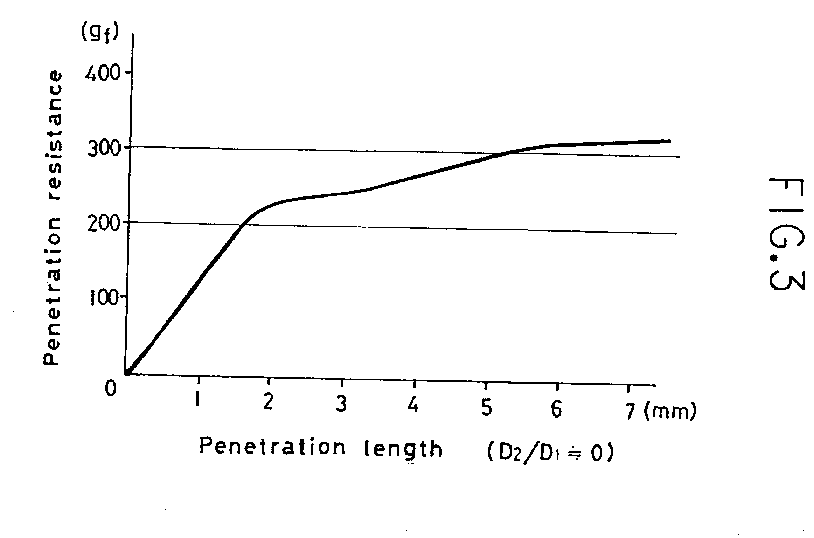

The results of experiments, in which the various needle points are used, are shown in Figs 3 to 7, which are corresponding to the needle points 2 in Figs. 2(a) to 2(e), respectively. A Porvair material sheet having thickness of1.1 mm is used for the measurement.

In Fig. 3, penetration resistance of the needle point 2 having a sharp tip P, currently ordinarily used, is shown by a curve indicating gradually increasing tendency. That is, the tendency of the curve indicates that the penetration resistance at a time that the surgical needle 1 enters into the specimen is extremely small and that the tip P is easily stuck even by weak force.

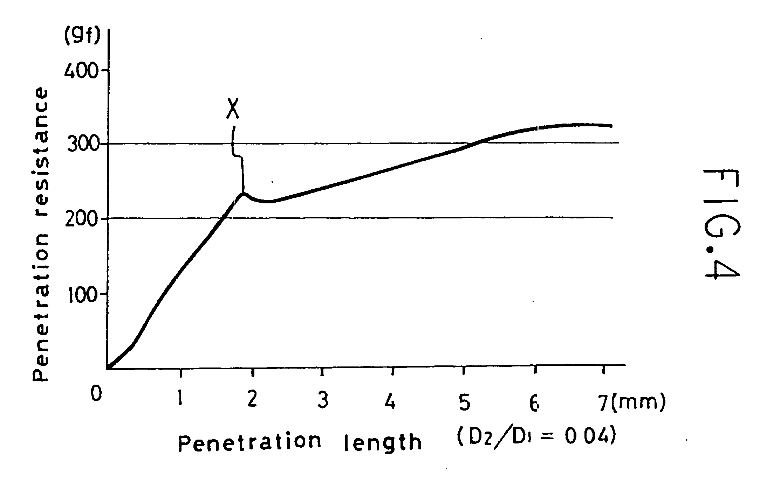

To the contrary, the graph in Fig. 4 has a peak X at a point where penetration length is 2 mm and keeps gradually increasing tendency with a pit of the penetration resistance right after passing the peak X. The peak X indicates penetration resistance at a time that the surgical needle 1 is stuck to the specimen. After the needle is stuck once, the resistance becomes low at one time, and then, the penetration resistance gradually increases in proportion to the penetration length of the needle point 2. In other words, the surgical needle 1 having the needle point 2 as of Fig. 2(b) does not enter into the specimen unless force of 220 gf is applied. Since the specimen is elastic and thereby, after a recess of about 2 mm is formed, the tip of the needle point 2 enters into there, the peak X comes to delay.

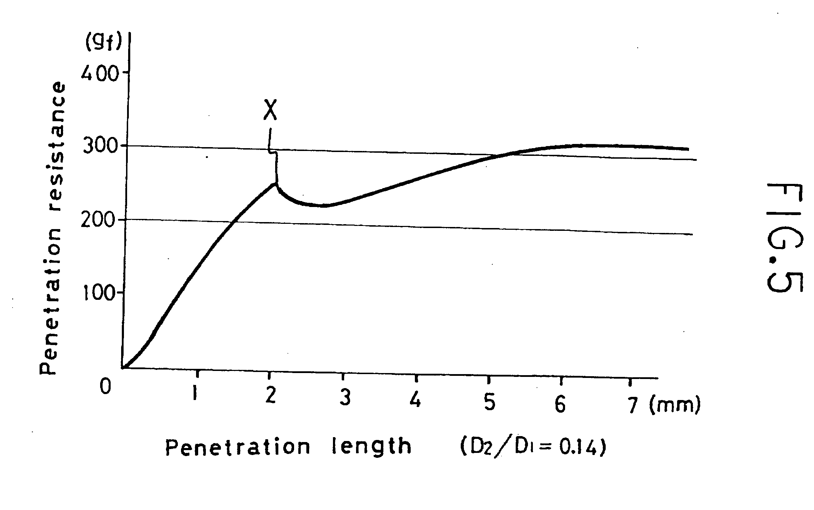

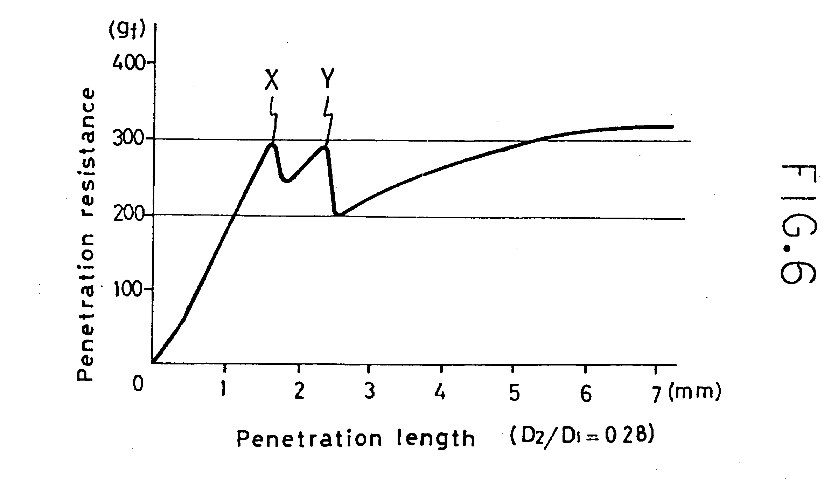

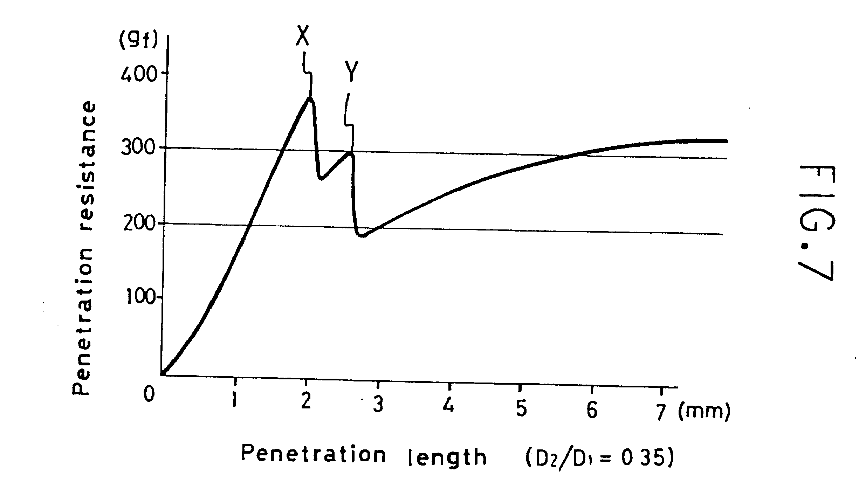

Fig. 5 indicates a higher peak X in its graph. That is, the graph indicates that the needle point 2 in Fig. 2(c) requires around 250 gf force to be entered into the specimen. Similarly, the peaks X in Figs. 6, 7 respectively indicate that the needle points 2 in Figs. 2(d), 2(e) require 290 gf and 380 gf, respectively, to be entered into the specimen. Although secondary peaks appear in Figs. 3, 7, this phenomenon is considered as an appearance of two peaks caused by penetration resistance that becomes higher where the needle point could not penetrate the surface layer and the flesh layer of the Porvair all at once.

On the basis of the result of the experiments described above, a shape of the needle point is determined so as not to stick easily and so as to have penetration resistance such that the needle could be smoothly handled. As shown in Fig. 2(a), in an ordinary surgical needle having a sharp tip T, the penetration resistance after stuck into the specimen is approximately 200 gf to 300 gf, and therefore, if the peak is within the range, the needle would be handled readily.

In the cases of Figs. 3(b), 3(c), and 3(d), peaks are produced within the aforementioned range. Judging from Figs. (d), (e), it is considered that the peak X would be 300 gf when the needle point has the blunt ratio of 0.30, and therefore, if the needle point has the blunt ratio of 0.30 or below, the surgical needle could be readily handled.

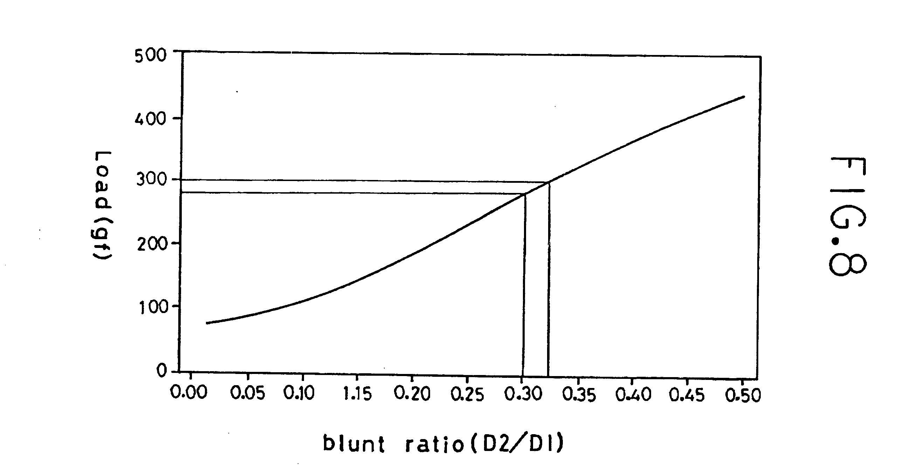

In addition, Fig. 8 shows a relation between blunt ratio (D2/D1) and peak X. Fig. 8 is a graph in which the abscissa indicates blunt ratio (D2/D1) and which the ordinate indicates load peak X at a time that the needle passes through the layer of Porvair, according to repeated experiments thus described. As shown in Fig. 8, if the blunt ratio is 0. 30, the load is equal to or less than 300 gf, and therefore, if the blunt ratio is equal to or less than the same value, the needle would be readily controlled.

As a result of another experiment in which a surgical needle 1 is penetrated through a rubber layer of thickness 0.2 mm, when the blunt ratio (D2/D1) was zero the load was 19 gf, and when the blunt ratio (D2/D1) was 0.04 the load was 51 gf. The rubber layer used in this experiment had the same quality and thickness as rubber gloves used for operations. Health providers handing the surgical needle 1 usually use thin medical rubber gloves. The surgical needle 1 is not readily, about two and a half times less, stuck into the rubber gloves when its blunt ratio is 0.04, in comparison with such an ordinary surgical needle 1. Accordingly, the needle point 2 of the blunt ratio of 0.04 has a shape capable of preventing the needle point 2 from sticking into their skin by mistake.

Consequently, according to the result of the experiments, setting the blunt ratio (D2/D1) to a range from 0.04 to 0.30 allows to constitute surgical needles 1 safe and easily handled.

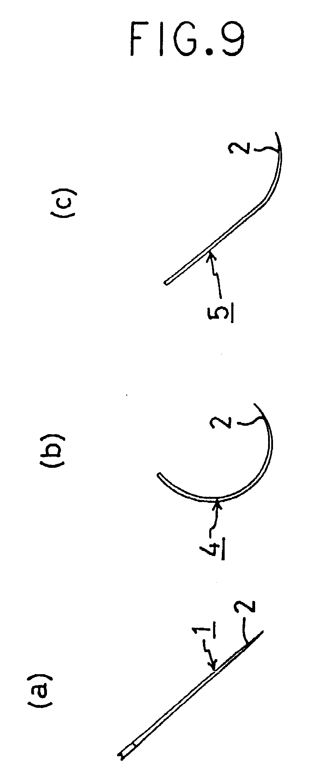

Figs 9(a) to 9(c) show examples of shapes of the needle points 2. Fig. 9(a) indicates a surgical needle 1 shaving a straight needle body 3. As shown in Figs. 9(b), 9(c), the needle point 2 could be, as a matter of course, formed in a shape as of a needle 4 having curvature or a needle 5 bent on the tip side. Moreover, in addition to formed in a round shape as shown in the embodiment, the tip of the needle point could be formed in a shape having an end face by cutting straight.

As described above, setting the ratio (D2/D1) of the diameter D2 of the needle point to the diameter D1 of the surgical needle body from 0.04 to 0.30 allows the surgical needle to be safe and readily handled, because the penetration resistance increases at a time that the needle sticks into the tissues and falls in a range allowing the needle to be readily handled at a time that the surgical needle passes through the tissues after the tip enters into the tissues.

It is understood that although the present invention has been described in detail with respect to preferred embodiments thereof, various other embodiments and variations are possible to those skilled in the art which fall within the scope and spirit of the invention, and such other embodiments and variations are intended to be covered by the following claims.

## Claims

1. A surgical needle comprising:
a body taperingly extending; and
a needle point provided at one end of said body and formed to be blunt so that the needle point does not enter into tissues unless a predetermined force or above is applied thereto,
wherein a ratio of diameter D2 of said needle point to diameter D1 of said body is set in a range between 0.04 and 0.30.

2. A surgical needle as set forth in claim 1, wherein said body has curvature.

3. A surgical needle as set forth in claim 1, wherein said body is straight.
